(19) Europäisches Patentamt
European Patent Office
Office européen des brevets

(11) Veröffentlichungsnummer: **0 004 918**
**B1**

(12) # EUROPÄISCHE PATENTSCHRIFT

(45) Veröffentlichungstag der Patentschrift: **23.09.81**

(51) Int. Cl.³: **C 07 D 233/60**, A 61 K 31/415

(21) Anmeldenummer: **79101051.5**

(22) Anmeldetag: **06.04.79**

(54) **Optisch aktives (-)-2-(2,4-Dichlorphenoxy)-1-(imidazol-1-yl)-4,4-dimethyl-pentan-3-on Verfahren zu seiner Herstellung sowie Arzneimittel, die diese Verbindung enthalten.**

(30) Priorität: **18.04.78 DE 2816818**

(43) Veröffentlichungstag der Anmeldung: **31.10.79 Patentblatt 79/22**

(45) Bekanntmachung des Hinweises auf die Patenterteilung: **23.09.81 Patentblatt 81/38**

(84) Benannte Vertragsstaaten: **BE CH DE FR GB LU NL SE**

(56) Entgegenhaltungen:
**DE-A-2 105 490**
**DE-A-2 242 454**
**DE-A-2 429 514**
**DE-A-2 455 954**
**DE-A-2 713 777**
**E. L. Eliel, Stereochemistry of Carbon Compounds, Mc Graw Hill 1962, Seiten 49 — 55**

(73) Patentinhaber: **BAYER AG, Zentralbereich Patente, Marken und Lizenzen, D-5090 Leverkusen 1, Bayerwerk (DE)**

(72) Erfinder: **Schmidt, Thomas, Dr., Wormser Strasse 23, D-5600 Wuppertal 1 (DE)**
Erfinder: **Krämer, Wolfgang, Dr., Am Eckbusch 39/162, D-5600 Wuppertal 1 (DE)**
Erfinder: **Büchel, Karl Heinz, Prof. Dr., Bergerheide 62, D-5600 Wuppertal 1 (DE)**
Erfinder: **Plempel, Manfred, Dr., Pahlkestrasse 5, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Optisch aktives (−)-2-(2,4-Dichlorphenoxy)-1-(imidazol-1-yl)-4,4-dimethyl-pentan-3-on, Verfahren zu seiner Herstellung sowie Arzneimittel, die diese Verbindung enthalten

Die vorliegende Erfindung betrifft das neue optisch aktive (−)-2-(2,4-Dichlorphenoxy)-1-(imidazol-1-yl)-4,4-dimethyl-pentan-3-on, ein Verfahren zu seiner Herstellung sowie Arzneimittel, insbesondere Antimykotika, die diese Verbindung enthalten.

Es ist bereits bekannt geworden, daß racemische 1-Ethylimidazole und deren Säureadditions-Salze, wie insbesondere das racemische 2-(2,4-Dichlorphenoxy)-1-imidazol-1-yl)-4,4-dimethyl-pentan-3-on-hydrochlorid, gute antimykotische Wirkung aufweisen (vergl. DE-OS 2 242 454 bzw. US-Patentschrift 3 940 413).

Gegenstand der Erfindung ist nun das neue optisch aktive (−)-2-(2,4-Dichlorphenoxy)-1-(imidazol-1-yl)-4,4-dimethyl-pentan-3-on der Formel

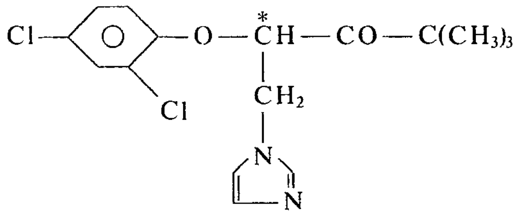

(I)

und seine physiologisch verträglichen Säureadditionssalze. Sie weisen starke antimykotische Eigenschaften auf.

Ein weiterer Gegenstand der Erfindung ist ein Verfahren zur Herstellung der neuen optisch aktiven Verbindung der Formel (I), welches darin besteht, daß man die entsprechende racemische Verbindung der Formel

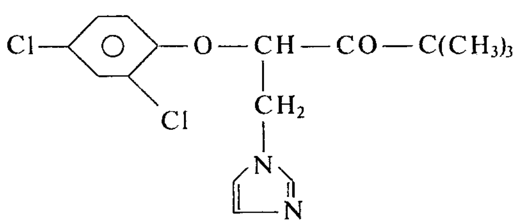

(II)

in einer ersten Stufe mit optisch aktiven Säuren, gegebenenfalls in Gegenwart eines Verdünnungsmittels, umsetzt, dann in einer zweiten Stufe die entstandenen Salze aufgrund ihrer unterschiedlichen Löslichkeit trennt, und danach in einer dritten Stufe die Verbindung der Formel (I) aus den entsprechenden Salzen mit Hilfe von Basen, gegebenenfalls in Gegenwart eines Verdünnungsmittels, in Freiheit setzt.

Überraschenderweise zeigt die erfindungsgemäße neue optische Antipode der Formel (I) eine bessere antimykotische Wirksamkeit als die bekannten entsprechenden racemischen 1-Ethyl-imidazole. Die erfindungsgemäßen Stoffe stellen somit eine wertvolle Bereicherung der Pharmazie dar.

Verwendet man 2-(2,4-Dichlorphenoxy)-1-(imidazol-1-yl)-4,4-dimethyl-pentan-3-on als Ausgangsstoff, L(+)-Weinsäure als optisch aktive Säure und wäßrige Natriumhydrogencarbonat-Lösung als freisetzende Base, so kann der Reaktionsablauf durch das folgende Formelschema wiedergegeben werden:

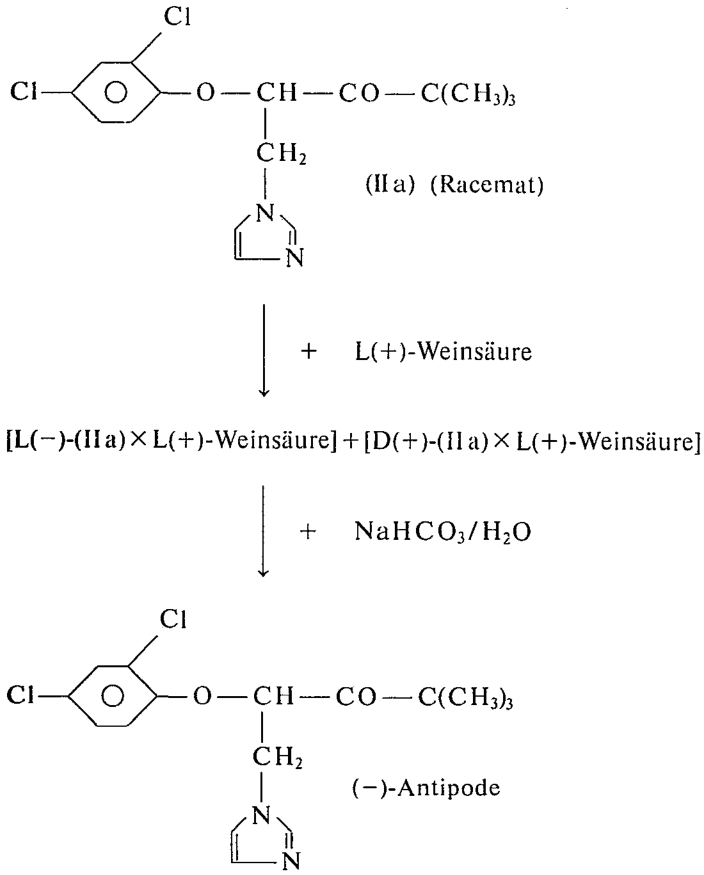


(II a) (Racemat)

[L(−)-(II a) × L(+)-Weinsäure] + [D(+)-(II a) × L(+)-Weinsäure]

(−)-Antipode

Das erfindungsgemäß verwendbare racemische Ausgangsprodukt der Formel (II) ist bekannt (vergleiche DE-A-2 242 454 bzw. US-Patentschrift 3 940 413). Es wird erhalten, indem man entsprechende 2-Halogenethyl- bzw. 2-Hydroxyethyl-ketone oder entsprechende 1-Halogenethylketone mit Imidazol in Gegenwart eines organischen Lösungsmittels, wie beispielsweise Acetonitril oder Toluol, bei Temperaturen zwischen 60 und 120° C umsetzt.

Bei der Durchführung des erfindungsgemäßen Verfahrens wird der Ausgangsstoff der Formel (II) in der ersten Stufe mit optisch aktiven Säuren umgesetzt. Als solche optisch aktiven Säuren können vorzugsweise verwendet werden: die D- bzw. L-Formen von Apfelsäure, Weinsäure, Diacetylweinsäure, Di-o-toluylweinsäure, Mandelsäure, Camphersäure, Camphersulfonsäure oder α-Bromcamphersulfonsäure. Die genannten optisch aktiven Säuren sind bereits bekannt.

Als Verdünnungsmittel kommen bei der Durchführung der ersten Stufe des erfindungsgemäßen Verfahrens alle inerten organischen Lösungsmittel in Frage. Hierzu gehören vorzugsweise Kohlenwasserstoffe wie Benzin, Benzol, Toluol und Xylol, halogenierte Kohlenwasserstoffe, wie Chloroform, Dichlormethan, Chlorbenzol und Dichlorbenzol, ferner Alkohole wie Äthanol und Methanol, weiterhin Ketone, wie Methyläthylketon oder Aceton, darüber hinaus Äther, wie Diäthyläther, Tetrahydrofuran oder Dioxan und Ester, wie Essigsäureäthylester.

Die Reaktionstemperaturen können bei der Durchführung der ersten Stufe des erfindungsgemäßen Verfahrens in einem größeren Bereich variiert werden. Im allgemeinen arbeitet man zwischen −20° C und 120° C, vorzugsweise zwischen 0° C und 60° C.

Bei der Durchführung der ersten Stufe des erfindungsgemäßen Verfahrens setzt man auf 1 Mol Ausgangsverbindung der Formel (II) vorzugsweise 0,5 bis 1 Mol an optisch aktiver Säure ein. Die Menge der Säure kann jedoch variiert werden, um die Bildung des diastereomeren Salzes der gewünschten optisch aktiven Base von vornherein zu begünstigen. Eine allgemein gültige Aussage läßt sich diesbezüglich nicht machen, denn von Fall zu Fall muß entschieden werden, in welcher Menge die Säure einzusetzen ist, da die Löslichkeit des jeweils entstehenden Salzes der gewünschten optisch aktiven Base nicht bei allen Derivaten gleich ist.

In der zweiten Stufe des erfindungsgemäßen Verfahrens erfolgt die Trennung der beiden diastereomeren Salze jeweils aufgrund der unterschiedlichen Löslichkeit durch fraktionierte Kristallisation.

In der dritten Stufe des erfindungsgemäßen Verfahrens wird die gewünschte optisch aktive (−)-Antipode der Formel (I) mit Hilfe von Basen in Freiheit gesetzt. Als Base kann vorzugsweise wäßriges Alkali, wie Natron- und Kalilauge oder wäßrige Natrium- und Natriumhydrogen-carbonat-Lösung, verwendet werden.

Als Lösungsmittel können bei der Durchführung der dritten Stufe des erfindungsgemäßen Verfahrens alle inerten organischen Lösungsmittel verwendet werden. Hierzu gehören vorzugsweise diejenigen organischen Lösungsmittel, die auch bei der Umsetzung in der ersten Stufe dieses Verfahrens vorzugsweise in Frage kommen.

Die Reaktionstemperaturen können bei der Durchführung der dritten Stufe des erfindungsgemäßen Verfahrens in einem größeren Bereich variiert werden. Im allgemeinen arbeitet man zwischen −20°C und 60°C, vorzugsweise zwischen 0°C und 30°C.

Bei der Durchführung der dritten Stufe des erfindungsgemäßen Verfahrens setzt man auf 1 Mol des betreffenden diastereomeren Salzes mindestens 2 Mol einer verdünnten wäßrigen Alkalilösung ein und überschichtet das Reaktionsgemisch zweckmäßigerweise mit einem in Wasser kaum löslichen organischen Lösungsmittel, wie zum Beispiel Äther. Die Isolierung des gewünschten optisch aktiven Antipoden der Base der Formel (I) erfolgt in der Weise, daß man nach beendeter Reaktion die organische Phase abtrennt, über Natriumsulfat trocknet, durch Abdestillieren des Lösungsmittels einengt und gegebenenfalls durch Umkristallisation von noch vorhandenem Racemat oder anderer optisch aktiver Antipode befreit.

Zur Herstellung von Säureadditionssalzen der Verbindung der Formel (I) kommen alle physiologisch verträglichen Säuren in Frage. Hierzu gehören vorzugsweise die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure sowie Sulfonsäure, wie z. B. p-Toluolsulfonsäure und 1,5-Naphthalindisulfonsäure.

Die Salze der Verbindung der Formel (I) können in einfacher Weise nach üblichen Salzbildungsmethoden, z. B. durch Lösen der Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäße Verbindung der Formel Formel (I) und ihre Säureadditions-Salze weisen antimikrobielle, insbesondere starke antimykotische Wirkungen auf. Sie besitzen ein sehr breites antimykotisches Wirkungsspektrum, insbesondere gegen Dermatophyten und Sproßpilze sowie biphasische Pilze, z. B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden:

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden:

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quartenäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyäthylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Äthylalkohol, Isopropylalkohol, Äthylcarbonat, Äthylacetat, Benzylalkohol, Benzylenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Sacharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen, und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirstoffe kann durch jeden Fachmann aufgrund seines Fachwissens leicht erfolgen.

Beispiel A

Antimykotische in-vitro-Wirksamkeit

Versuchsbeschreibung:

Die in-vitro-Prüfungen wurden im Reihenverdünnungstest mit Keiminokula von $10^{-3}$ Keimen/ml Substrat bei Hefen und $10^{-4}$ Keimen/ml Substrat bei Dermatophyten durchgeführt. Als Nährmedium diente Yeast-Nitrogen-Base, ein allgemein bekanntes Nährsubstrat, das von der Firma Difco unter der Nummer: Difco-Nr. 0392-15 vertrieben wird.

Die Bebrütungstemperatur betrug 28° C; die Bebrütungsdauer lag bei 48 – 72 Stunden.

In diesem Test zeigt die erfindungsgemäße optisch aktive (–)-Antipode eine bessere mykotische Wirkung als das Racemat und als die andere optisch aktive Antipode.

Die Resultate dieses Tests sind in einer Anlage mit Datum 11. Sept. 1980, enthalten. Diese Anlage ist der Öffentlichkeit gemäß Art. 128 3.) und 4.), EPÜ zugänglich.

Beispiel 1

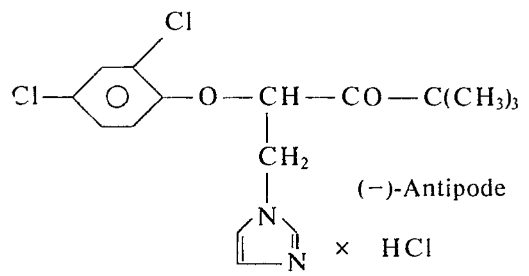

(–)-Antipode

1. Stufe

Darstellung des Weinsäuresalzes

34 g (10,1 Mol) 2-(2,4-Dichlorphenoxy)-1-(imidazol-1-yl)-4,4-dimethyl-pentan-3-on und 15 g (0,1 Mol) L(+)-Weinsäure werden in 50 ml warmem Äthanol gelöst und filtriert. Beim Abkühlen kristallisiert das Tartrat aus. Der entstandene Kristallkuchen wird abgesaugt und getrocknet. Man erhält 47 g (96% der Theorie) an diastereomerem Tartrat vom Schmelzpunkt 142° C.

2. Stufe

Trennung der diastereomeren Tartrate

Man löst 47 g (0,096 Mol) Tartrat in heißem n-Butanol, filtriert und läßt drei Tage bei Raumtemperatur auskristallisieren. Der ausgefallene Feststoff wird abgesaugt (26,8 g Fp: 136 – 142° C) und abermals aus heißem n-Butanol umkristallisiert. Diese Prozedur wird mehrfach wiederholt. Aus den jeweils anfallenden Mutterlaugen kristallisieren weitere, unreinere Fraktionen an schwerer löslichem Tartrat, die gesammelt und separat umkristallisiert werden. Der dabei anfallende Feststoff wird jeweils dem schwerer löslichen Salz zugeschlagen, während die Mutterlauge mit denen der primären Kristallisationen vereinigt wird.

Durch diese Verfahrensweise erhält man schließlich einen Feststoff, in dem das schwerer lösliche Tartrat angereichert ist, das die (+)-Antipode enthält, sowie eine Mutterlauge, in der sich der überwiegende Teil des leichter löslichen Tartrats befindet, das die (–)-Antiopode enthält.

Man saugt die Mutterlauge zur Trockne ein und erhält 17,3 g (73,5% der Theorie) Tartrat der (–)-Antipode des 2-(2,4-Dichlorphenoxy)-1-(imidazol-1-yl)-4,4-dimethyl-pentan-3-ons vom Schmelzpunkt 132 – 137° C.

3. Stufe

Darstellung der (–)-Antipode

In einem 250-ml-Scheidetrichter werden 33 g (0,069 Mol) Tartrat der (–)-Antipode in 300 ml Äther aufgeschlämmt und mit gesättiger, wäßriger Bicarbonatlösung versetzt.

Man schüttelt so lange, bis alles gelöst ist, trennt die wäßrige Phase ab und wäscht die organische Phase noch einmal mit 20 ml Wasser. Nach Trocknen über Natriumsulfat und Abziehen des Lösungsmittels wird das zurückbleibende Öl in etwas warmem, absolutem Äther aufgenommen und noch enthaltenes Racemat durch Zugabe eines Impfkristalls zuerst bei Raumtemperatur und dann bei 0°C auskristallisiert. Nach Filtration und Abziehen des Äthers erhält man die (−)-Antipode des 2-(2,4-Dichlorphenoxy)-1-(imidazol-1-yl)-4,4-dimethyl-pentan-3-ons, die mit 50 ml ätherischer Salzsäure versetzt und so lange gerührt wird, bis alles gelöst ist. Danach dampft man zur Trockne ein, nimmt den Rückstand in warmem Essigester auf und läßt auskristallisieren. Man erhält 6 g (23,1% der Theorie) der (−)-Antipode des 2-(2,4-Dichlorphenoxy)-1-(imidazol-1-yl)-4,4-dimethyl-pentan-3-on-hydrochlorids vom Schmelzpunkt 160−162°C mit einem Drehwinkel von $[\alpha]_{589}^{D} = -39{,}4°$ (5%ige Lösung in Äthanol).

## Patentansprüche

1. Optisch aktives (−)-2-(2,4-Dichlorphenoxy)-1-(imidazol-1-yl)-4,4-dimethyl-pentan-3-on der Formel (I)

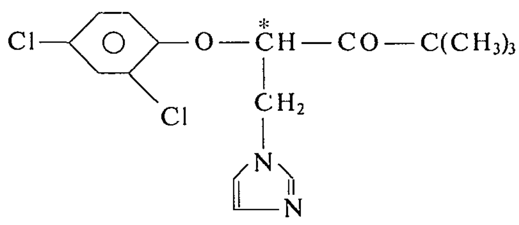

(I)

und seine physiologisch verträglichen Säureadditionssalze.

2. Verfahren zur Herstellung von (−)-2-(2,4-Dichlorphenoxy)-1-(imidazol-1-yl)-4,4-dimethyl-pentan-3-on der Formel (I), dadurch gekennzeichnet, daß man die racemische Verbindung der Formel (II)

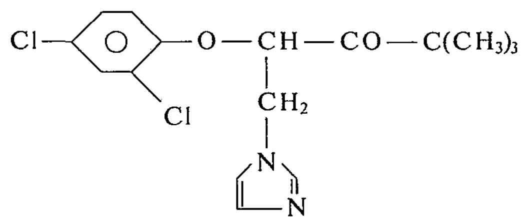

(II)

in einer ersten Stufe mit optisch aktiven Säuren, gegebenenfalls in Gegenwart eines Verdünnungsmittels umsetzt,
dann in einer zweiten Stufe die entstandenen Salze aufgrund ihrer unterschiedlichen Löslichkeit trennt, und
danach in einer dritten Stufe die optische (−)-Antipode der Verbindung der Formel (I) aus den entsprechenden Salzen mit Hilfe von Basen, gegebenenfalls in Gegenwart eines Verdünnungsmittels, in Freiheit setzt.

3. Arzneimittel, gekennzeichnet durch einen Gehalt an der optischen Antipode gemäß Anspruch 1.

## Claims

1. Optically acitve (−)-2-(2,4-dichlorophenoxy)-1-(imidazol-1-yl)-4,4-dimethyl-pentan-3-one of the formula (I)

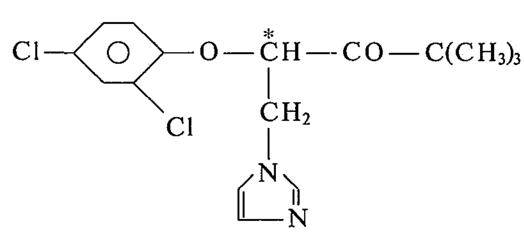

(I)

and its physiologically acceptable acid addition salts.

2. Process for the production of ( – )-2-(2,4-dichlorophenoxy)-1-(imidazol-1-yl)-4,4-dimethyl-pentan-3-one of the formula (I), characterised in that the racemic compound of the formula (II)

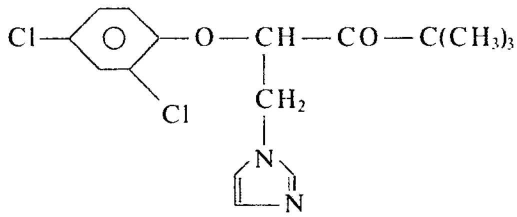

(II)

is reacted with optically active acids in a first stage, optionally in the presence of a diluent, then, in a second stage, the salts formed are separated on the basis of their different solubility, and thereafter, in a third stage, the optical ( – )-antipode of the compound of the formula (I) is liberated from the corresponding salts with the aid of bases, optionally in the presence of a diluent.

3. Medicaments characterised in that they contain the optical antipode according to Claim 1.

## Revendications

1. La ( – )-2-(2,4-dichlorophénoxy)-1-(imidazole-1-yl)-4,4-diméthylpentane-3-one-énantiomère de formule (I)

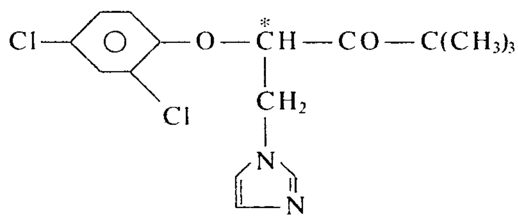

(I)

et ses sels formés par addition avec des acides acceptables pour l'usage pharmaceutique.

2. Procédé de préparation de la ( – )-2-(2,4-dichlorophénoxy)-1-(imidazole-1-yl)-4,4-diméthylpentane-3-one de formule (I), caractérisé en ce que, dans un premier stade, on fait réagir le composé racémique de formule (II)

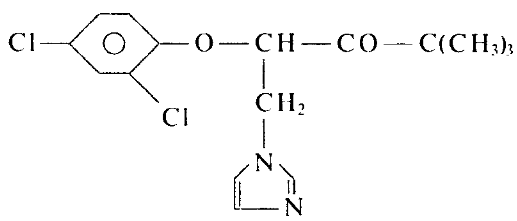

(II)

avec des acides énantiomères, le cas échéant en présence d'un diluant; dans un deuxième stade, on sépare les sels obtenus sur la base de leurs solubilités différentes; et, dans un troisième stade, on libère l'antipode optique ( – ) du composé de formule (I) à partir des sels correspondants à l'aide de bases, le cas échéant en présence d'un diluant.

3. Médicament caractérisé en ce qu'il contient l'antipode optique selon la revendication 1.